# EUROPEAN PATENT APPLICATION

(11) **EP 4 439 579 A1**
(43) Date of publication of application: **02.10.2024**
(21) Application number: 24166410.1
(22) Date of filing: 26.03.2024
(51) Int. Cl.: G16H 30/20, G16H 30/40

(54) **IMAGE VIEWING PIPELINE FOR MEDICAL IMAGE VIEWER**

(30) Priority: 27.03.2023 US 202363492350 P
(71) Applicant: Tempus AI, Inc., Chicago, IL 60654 (US)
(72) Inventor: LOWE, Madison Ragna, Okotoks, Alberta T1S 1P1 (CA); FRANZ, Cole Sawyer, Calgary, Alberta T3H 3R4 (CA); HUGHES, Matthew Charles, Calgary, Alberta T3L 3C6 (CA)
(74) Representative: Eisenführ Speiser

(57) **Abstract**

An image viewing pipeline is provided as an independently releasable component of an image viewing application. Images are provided for viewing in the application using dedicated infrastructure for the image viewing pipeline that is separate from infrastructure used to provide other functionality of the image viewing application. The image viewing pipeline receives images from a DICOM image service and provides the images to a client application. The client application is operative to request images, process images, and cache images. The client application is further operative to satisfy individual image requests and may prefetch stacks of images using the image viewing pipeline.

## Description

### BACKGROUND

### Technical Field

The present disclosure relates generally to the field of medical imaging, and more particularly, to web-based medical image viewers. More specifically, the present disclosure is directed to an image viewing pipeline and related components for web-based medical image viewers.

### Description of the Related Art

In recent years, the field of medical imaging has seen tremendous advancements in the development of high-quality digital imaging technology. With the advent of web-based platforms, there is an increasing need for medical professionals to have access to these images from anywhere at any time. Medical image viewers are essential tools that enable healthcare professionals to visualize, analyze, and interpret these images to provide accurate diagnoses and treatment plans.

But the development of high-quality imaging technology has led to a commensurate increase in the size and complexity of medical images. This presents significant challenges for web-based medical image viewers. Large image files require significant processing power and storage capacity, while the need to transmit images across networks can result in significant latency and reduced image quality. Additionally, medical images may contain sensitive patient data, necessitating the implementation of robust security measures to protect patient privacy.

Existing web-based medical image viewers have attempted to address some of these challenges by using various image compression techniques; however, these techniques often result in loss of image quality or introduction of significant visual artifacts. Furthermore, existing viewers may not be capable of handling large volumes of data or allow for efficient image processing and analysis, which are critical for accurate diagnosis and treatment.

### BRIEF DESCRIPTION OF THE SEVERAL VIEWS OF THE DRAWINGS

Figure 1 shows a screenshot of an example graphical user interface (GUI) for a web-based medical image viewer, in accordance with embodiments described herein.
Figure 2 shows an example block diagram of an image viewing pipeline in accordance with embodiments described herein.
Figures 3A, 3B, and 3C are block diagrams illustrating a staged deployment of an image viewing pipeline in accordance with embodiments described herein.
Figure 4 is a flow diagram illustrating a process used to provide an image viewing pipeline in accordance with embodiments described herein.
Figure 5 is a flow diagram illustrating image prefetch for an image viewing pipeline in accordance with embodiments described herein.
Figure 6 is a flow diagram that shows a process for retrieving pixel data from image data received from a medical image service in accordance with embodiments described herein.
Figure 7 is a flow diagram that shows a rendering pipeline of the image viewing pipeline in accordance with embodiments described herein.
Figure 8 shows a screenshot of an example graphical user interface (GUI) for a web-based medical image viewer in which two co-registered studies are presented to a user, in accordance with embodiments described herein.
Figure 9 shows a screenshot of an example graphical user interface (GUI) for a web-based medical image viewer, in which two co-registered studies are presented to a user in an enlarged portrait mode, in accordance with embodiments described herein.
Figure 10 is a block diagram illustrating elements of an example computing device utilized in accordance with embodiments described herein.

### DETAILED DESCRIPTION

In the following description, certain specific details are set forth in order to provide a thorough understanding of various disclosed implementations. However, one skilled in the relevant art will recognize that implementations may be practiced without one or more of these specific details, or with other methods, components, materials, etc. In other instances, well-known structures associated with computer systems, server computers, and/or communications networks have not been shown or described in detail to avoid unnecessarily obscuring descriptions of the implementations.

Unless the context requires otherwise, throughout the specification and claims that follow, the word "comprising" is synonymous with "including," and is inclusive or open-ended (i.e., does not exclude additional, unrecited elements or method acts).

Reference throughout this specification to "one implementation," "an implementation," "some implementations," or "various implementations" means that a particular feature, structure, or characteristic described in connection with the implementation is included in at least one implementation. Thus, the appearances of the phrases "in one implementation," "in an implementation," "in some implementations," or "in various implementations" in various places throughout this specification are not necessarily all referring to the same implementation or implementations. Furthermore, the particular features, structures, or characteristics may be combined in any suitable manner in one or more implementations.

As used in this specification and the appended claims, the singular forms "a," "an," and "the" include plural referents unless the context clearly dictates otherwise. It should also be noted that the term "or" is generally employed in its sense including "and/or" unless the context clearly dictates otherwise.

Radiologists and other healthcare providers often analyze medical images to diagnose patients, recommend treatment plans, etc. To perform certain workflows, radiologists need to scroll through images quickly and reliably. As an example, lung computed tomography (CT) images will often come as a stack of 500 images of 512x512 pixel size. The radiologist needs to scroll through the images quickly and smoothly, and the images need to be viewed in full quality. To provide this functionality, in at least some implementations, the images of a series may be sent from the cloud to a client device. The images of the series may then be cached on the client device to provide rapid access to the images. But in at least some implementations, the download speed is limited (e.g., to about a max of 70 Mbps download speed), and much higher performance (e.g., up to 500Mbps or more) is desirable if the bandwidth of the user's client device allows for such speeds.

Further, viewing images is critical functionality for radiologists and other healthcare providers. The ability to view images is often more important than the ability to make measurements or take screenshots, since radiologists can sometimes make diagnoses solely using images. Therefore, it is important that changes and improvements to a medical image viewer that are released do not break the image viewing functionality.

The inventors of the present disclosure have identified a need for a more efficient and robust image viewing pipeline (IVP) for web-based medical image viewers that can handle large volumes of data, provide high-quality images, and ensure patient data security. Such IVP significantly improves the accuracy and speed of medical image analysis, leading to better patient outcomes.

In some implementations, first graphical user interface (GUI) components of a GUI of a web-based medical viewer application are hosted by a first server. The first GUI components include images to be presented in a central viewing area of the GUI of the web-based medical viewer application. Second GUI components of the GUI are hosted by a second server different from the first server. The second GUI components include GUI components of the application other than the first GUI components. The first server serves the GUI components to a client device for rendering the GUI to a user of the client device. The second server serves the second GUI components to the client device for rendering the GUI to the user of the client device.

Because a separate server is used to host images, other features of the application may be decommissioned or updated, such that critical image-viewing capabilities are maintained even if some GUI components are unavailable. Images may be viewed independently of other GUI components because delivery is handled by dedicated central image viewing infrastructure instead of general application infrastructure.

In some implementations, the image viewing functionality is further isolated into its own repository, and its own reusable client package. This allows for imaging viewing pipeline updates to be pushed separately and in a lower-risk way than the rest of the application. Other application features may be tested in a more targeted way, such that general application infrastructure failures are unlikely to impact the image viewing pipeline.

In some implementations, relevant medical images are preemptively requested when a user loads a study, and the relevant medical images are retrieved in the background and cached in the client browser. In various medical workflows, such as reviewing images of lungs, users need to be able to scroll through an entire stack of hundreds of full-quality chest CT images. The CT images should be rendered quickly and smoothly, to allow the user to compare various images in the set. In some implementations, various parallel processing techniques such as multithreading, graphics processing unit (GPU) rendering, etc., may be used to improve performance of image caching, rendering, or both.

In some implementations, separating image viewing pipeline components from other application components may be facilitated by using parameters that indicate various URLs of services used by the application to enable communication between the services. For example, the image viewing pipeline may retrieve images from a medical image service. The parameters are stored using a parameter storage service and may be created or updated at any time, to change stack dependencies.

In some implementations, staged deployments of application updates are used to minimize the chances of the image viewing pipeline becoming inoperable. Changes to the image viewing functionality may be released to users without breaking or interrupting functionality of the image viewing pipeline. Two or more versions of the image viewing pipeline may be deployed side-by-side. A portion of traffic is then routed to a new version, and a portion of traffic is routed to an old version. Performance of the new version is monitored for issues before all traffic is increasingly routed to the new version.

In some implementations, source-controlled and code-reviewed tooling for deploying and managing the central image viewer infrastructure is used to improve its stability. IVP tooling is written as bash scripts which are executed from an established tooling system. The bash scripts provide manual pages and tab completion to ensure the IVP tooling complies with established code-review processes.

In some implementations, a comprehensive versioning interface is provided by which a user may view a version of each service used by the application.

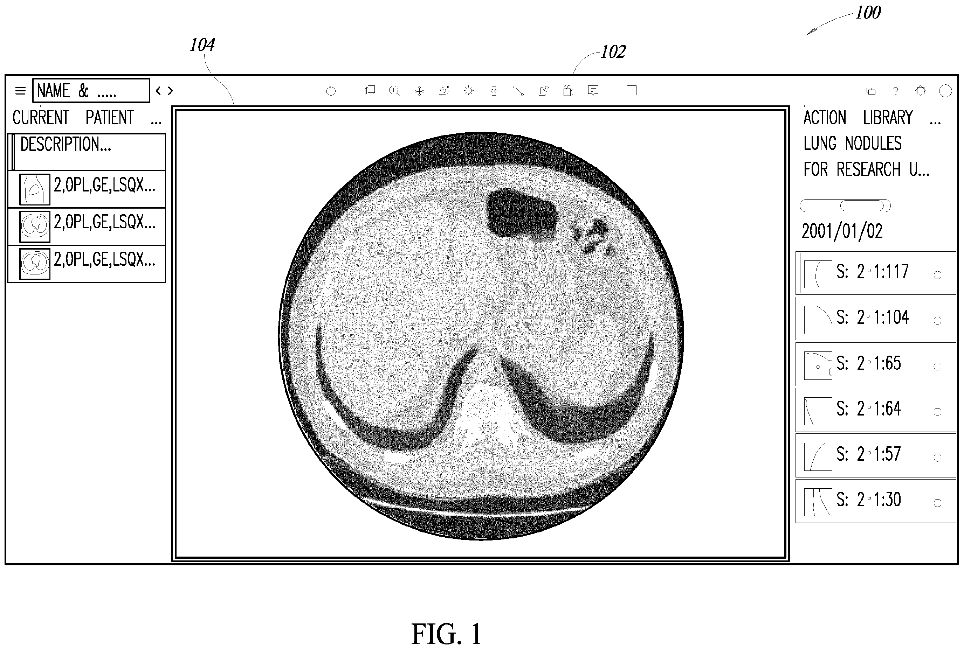

Figure 1 shows a screenshot of an example graphical user interface (GUI) 100 for a web-based medical image viewer (i.e., the "application"), according to one non-limiting illustrated implementation. As discussed in detail below, the GUI includes a central image viewer 104 that is a distinct GUI component. Central image viewer 104 is displayed using assets sourced from one or more servers that are separate from the servers used to source assets for GUI components 102 shown in GUI 100. The central image viewer 104 may therefore function regardless of a status of the GUI components 102. Although the central image viewer 104 is referred to as a "central" image viewer, the central image viewer 104 may be located anywhere in the GUI.

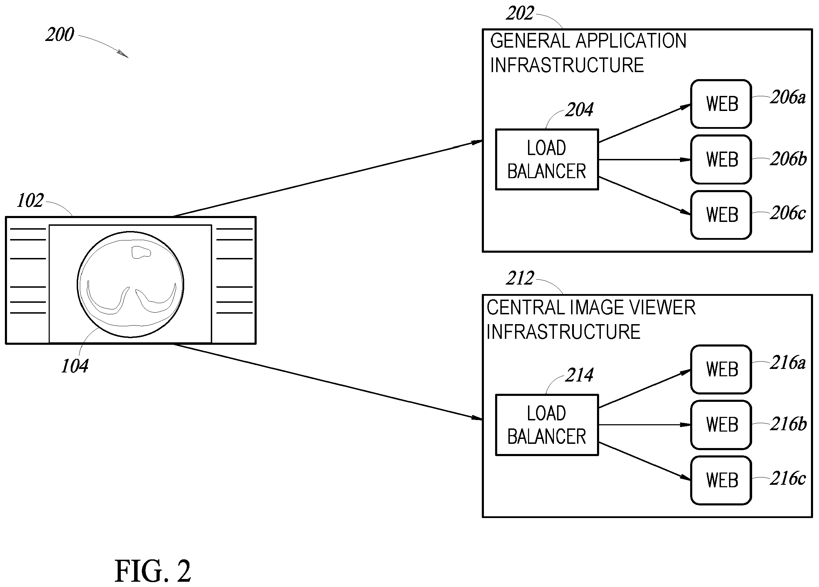

Figure 2 shows an example block diagram of an environment 200 in which an image viewing pipeline operates according to one non-limiting illustrated implementation. GUI components 102 are served using general application infrastructure 202, which includes a load balancer 204 to distribute load incurred via the GUI components 102 to servers 206a, 206b, and 206c. In various implementations, the load balancer 204 may distribute the load incurred by the GUI components 102 to any number of servers.

Central image viewer 104 is served using central image viewing infrastructure 212, which includes load balancer 214 to distribute load incurred via the central image viewer 104 to server 216a, server 216b, and server 216d. In various implementations, the load balancer 214 may distribute the load incurred by the central image viewer 104 to any number of servers.

As discussed herein, displaying series of medical images via central image viewer 104 may require significant bandwidth. In conventional image viewing pipelines, a server that serves medical images via central image viewer 104 also servers other GUI components 102.

In implementations described herein, performance at scale is improved because the central image viewer is served using separate servers from the rest of the application. The image viewing pipeline maintains reliable performance from central image viewing infrastructure 212 even if the general application infrastructure 202 experiences excess load or degraded performance. To support this functionality, general application infrastructure 202 may be used to both discover and provide a URL and an authentication token by which the application may access the central image viewing infrastructure 212.

During application startup, the application queries general application infrastructure 202 for configuration information regarding the central image viewing infrastructure 212. The configuration information may include an endpoint or port of the central image viewing infrastructure 212, or any other client-specific configuration relevant to procuring medical images from the central image viewing infrastructure 212 such as a number of concurrent images to request at a time, as detailed below. The general application infrastructure 202 may gather the configuration information from a parameter manager such as Amazon Web Services ParameterStore^{®}, HashiCorp Vault^{®}, etc., and returns the configuration information to the client device, as well as a method to retrieve authentication information for the client device to connect and authenticate to the central image viewing infrastructure 212. In at least some implementations, the authentication information is passed from the general application infrastructure 202 to a component of the application that is associated with the GUI components 102, then to a component of the application that is associated with the central image viewer 104, and then to the central image viewing infrastructure 212. In some implementations, the component of the application that is associated with the central image viewer 104 may store authentication information such that authentication may be performed without receiving the authentication information via the general application infrastructure 202 or the component of the application that is associated with GUI components 102. For example, the authentication information may be passed directly from the general application infrastructure 202 to the central image viewing infrastructure 212. In at least some implementations, the same URL and configuration to access the central image viewing infrastructure 212 are used for the duration of the user's session, but the authentication token only lasts for a particular duration (e.g., 30 minutes) before it is refreshed.

Availability of the requested medical images is improved because the central image viewing infrastructure 212 is substantially isolated from general application infrastructure 202 and is not likely to be affected when various other aspects of the application such as other GUI components 102 are changed. As a result, developers of the medical image viewer may develop and test the image viewing pipeline separately from the rest of the system. For example, the developers may modify and potentially break a pipeline for delivering GUI components besides the images without jeopardizing the image viewing pipeline.

Additionally, the image viewing pipeline itself may experience failures or service errors. Several conditions may generate a service error in the image viewing pipeline. For example, the image viewing pipeline may fail to fetch a raw image from the medical image service within a maximum number of allowed attempts. Logging a web socket associated with the image viewing pipeline longer than a configured time limit may also produce a service error in the image viewing pipeline. In general, various non-recoverable errors may arise in operation of the general application infrastructure 202 or the central image viewing infrastructure 212. Functionality of the image viewing pipeline may be maintained using one or more redundant image viewing pipelines within or side-by-side central image viewing infrastructure 212, or using a shared pipeline within general application infrastructure 202.

An example implementation of an Image Viewing Pipeline (IVP) is discussed below. It should be appreciated that variations may be made to embodiments disclosed herein without departing from the scope of the present disclosure.

The Image Viewing Pipeline is an independently releasable service designed to optimize image delivery and processing. The IVP may include server-side and client-side components. Referring to Figure 2, the term "IVP" may refer to functionality performed by a client device, the central image viewing infrastructure 212, etc., or a combination thereof used to provide medical images.

The central image viewing infrastructure 212 is in communication with a Digital Imaging and Communications in Medicine (DICOM) image service from which medical images are sourced. In some implementations, communication between the central image viewer infrastructure 104 and the client device is accomplished using web sockets. The central image viewing infrastructure 212 receives image requests from the client device. The central image viewing infrastructure 212 then fulfills the image requests by acquiring image data from the DICOM image service and sending the image data to the client device, which may process the image data, cache it, or both.

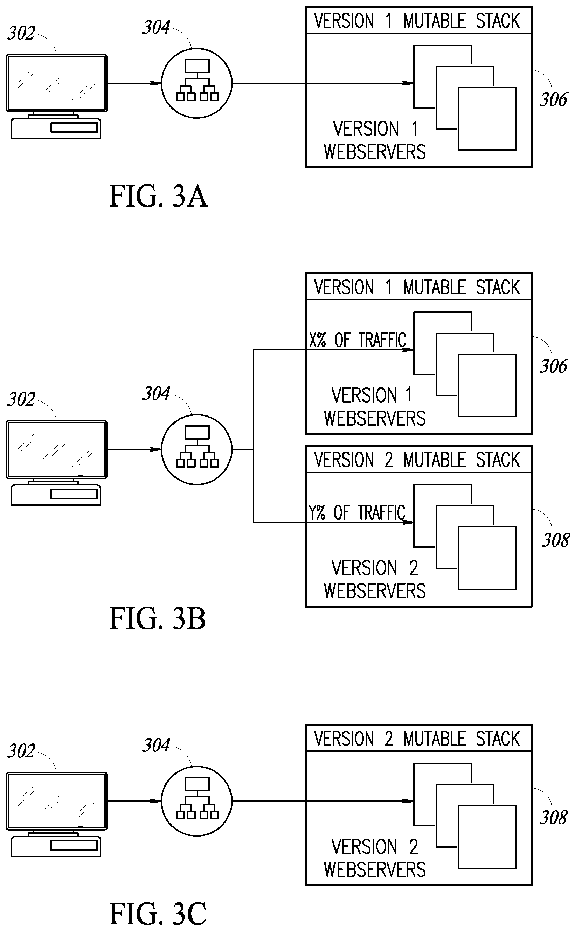

Figures 3A-3C are block diagrams of an example infrastructure for deploying zero-downtime updates to an image viewing pipeline in accordance with embodiments described herein. New versions of the IVP may be released with zero downtime, further enabling uninterrupted performance of the central image viewer. This may be achieved by providing multiple concurrent versions of the IVP.

Figure 3A depicts a first version of the IVP in typical operation before a zero-downtime release. Load balancer 304 facilitates communication between the client device 302 and first IVP infrastructure 306, which comprises resources such as the central image viewing infrastructure and the general application infrastructure that are used to support the first version of the IVP.

Figure 3B depicts a zero-downtime release of a second version of the IVP, whereby traffic is gradually routed away from first IVP infrastructure 306 and to second IVP infrastructure 308. In some implementations, when a change to the IVP is to be made, the second IVP infrastructure 308 reflecting the change is brought into operation while the first IVP infrastructure 306 remains operable and continues to service IVP traffic, such as communications with client device 302. Load balancer 304 reroutes a portion of IVP traffic to the second IVP infrastructure 308. Typically, the portion of rerouted traffic is small, such as 1%, 5%, or 10%, of total IVP traffic such that service is not significantly degraded if the second IVP infrastructure 308 experiences failures or other adverse issues.

If the second IVP infrastructure 308 meets performance standards, load balancer 304 may incrementally increase the portion of IVP traffic that it directs to the second IVP infrastructure. For example, the portion of traffic routed to the second IVP infrastructure 308 may be increased by 1%, 5%, or 10% of total IVP traffic. If performance standards continue to be met as load on the second IVP infrastructure 308 is increased, load balancer 304 may continue to incrementally increase the portion of IVP traffic that it routes to the second IVP infrastructure 308 until all IVP traffic is routed to second IVP infrastructure. The portion of IVP traffic that is routed to a given IVP infrastructure may be controlled via listener rules on the load balancer 304. Availability of the medical images is improved because, if the medical images cannot be provided to client device 302 using the second IVP infrastructure 308, the medical images may be provided using the first IVP infrastructure 306.

Figure 3C depicts the second version of the IVP in typical operation after the zero-downtime release is completed. Load balancer 304 facilitates communication between client device 302 and the second IVP infrastructure 308. In Figure 3C, the load balancer 304 routes no IVP traffic to the first IVP infrastructure 306. In some implementations, the first IVP infrastructure 306 may remain operational such that load balancer 304 may reroute a portion of IVP traffic to the first IVP infrastructure 306 on demand. If, for example, performance of second IVP infrastructure 308 degrades, traffic may be routed to first IVP infrastructure 306 with little or no interruption of service. The first IVP infrastructure 306 may be taken out of operation when the second IVP infrastructure 308 meets performance requirements that may include a threshold uptime, error count, response time, etc. In some implementations, a configuration of first IVP infrastructure 306 is stored such that the first IVP infrastructure 306 may be made operational when desired.

An IVP infrastructure is typically portable and may be deployed using various techniques. In some implementations, the IVP infrastructure such as the first IVP infrastructure 306 comprises a distributed application. For example, the IVP infrastructure may be implemented using a container image and operated as an autoscaling group of a compute service such as Amazon EC2^{®}.

In some implementations, infrastructure for load balancer 304 is implemented using a stack that is separate from that of other IVP infrastructure.

In some implementations, the IVP infrastructure is deployed using a cloud deployment manager such as Amazon AWS Cloud Development Kit^{®} (CDK) or Google Cloud Platform^{®} (GCP) deployment manager.

As discussed herein, the IVP infrastructure typically includes various services that may be implemented using various servers. For example, central image viewing infrastructure 212 communicates with a DICOM medical image service to procure medical images. In some implementations, parameters for locating and accessing the various services comprising the IVP are stored using a parameter storage service such as Amazon Web Servers ParameterStore^{®}, HashiCorp Vault^{®}, etc. By storing information to discover various services in the IVP infrastructure in a parameter storage service, computing resources may be added to the IVP infrastructure with little configuration.

In some implementations, a version number for the application is created based on a version number of the IVP and version numbers of the various services with which the IVP interacts, such as the DICOM image service. The latest version may be stored in the parameter storage service and retrieved at runtime to be displayed to the user. Thus, several versions of the application may be deployed at the same time, and a version number of the version of the application currently being used is displayed to the user.

In some implementations, the user logging into the application prompts a versioning reporting process whereby a version number of each service used by the application is obtained from each service. The version numbers are then displayed to the user. In some implementations, the version numbers are verified against published version numbers of the services to ensure the version numbers are supported. Reliable versioning is often critical. For example, regulatory approval from agencies such as the U.S. Food and Drug Administration may involve compliance with a schema for versioning of approved data products, and display of versioning information to the user.

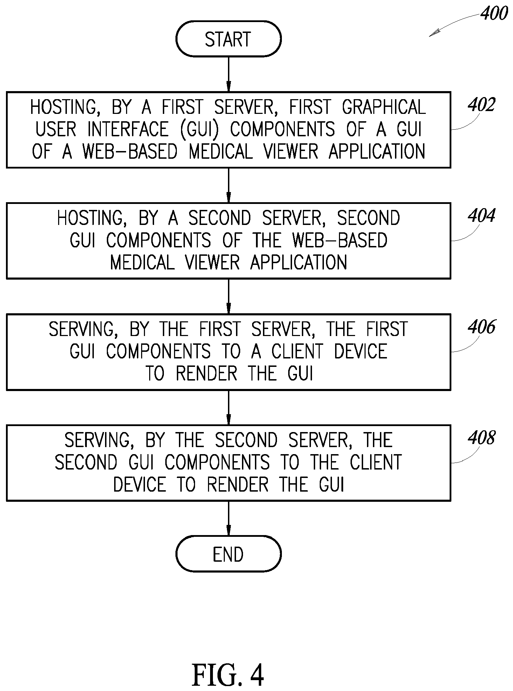

Figure 4 is a flow diagram illustrating a process 400 used to provide an image viewing pipeline in accordance with embodiments described herein.

Process 400 begins, after a start block, at block 402, where a first server hosts first graphical user interface (GUI) components of a GUI of a web-based medical viewer application. As discussed herein, the first GUI components typically include images to be displayed in a central image viewer of the GUI. For example, the first GUI components may include images from one or more co-registered medical studies. After block 402, process 400 continues to block 404.

At block 404, a second server hosts second GUI components of the GUI of the web-based medical viewer application. The second GUI components may include various measuring tools, navigation interfaces, options, menus, etc. In some implementations, the second GUI components include all GUI components that are not the first GUI components. After block 404, process 400 continues to block 406.

At block 406, the first server serves the first GUI components to a client device to render the GUI. In some implementations, images constituting the first GUI components are sent to the client device in response to detecting that the client device has loaded a medical study associated with the images. After block 406, process 400 continues to block 408.

At block 408, the second server serves the second GUI components to the client device to render the GUI. In some implementations, the first GUI components and the second GUI components are rendered concurrently. After block 408, process 400 ends at an end block.

In various implementations, at least one of the first server and the second server are implemented using a container. For example, the first server may be run in a Docker^{®} container.

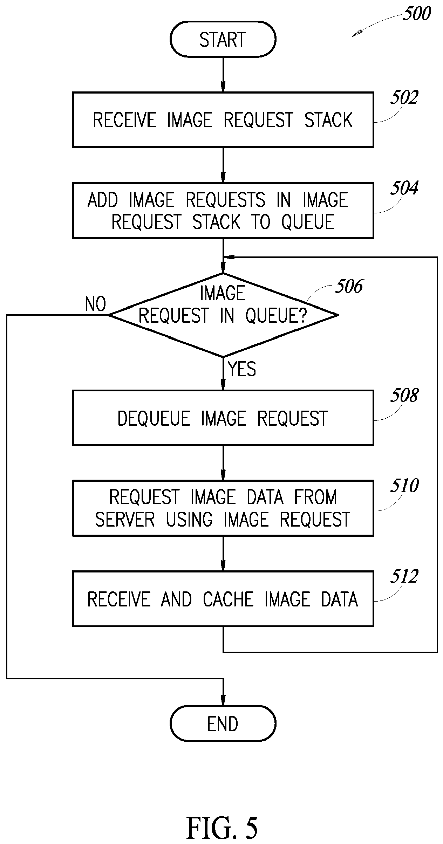

Figure 5 illustrates a flow diagram of a method for prefetching an image stack in the IVP, according to one non-limiting illustrated implementation. As discussed herein, prefetching images from a medical image service when a medical study that includes the images is opened may reduce latency when a user views the images.

Process 500 begins, after a start block, at block 502, where an image request stack is received. In some implementations, image requests in the image request stack may be used to request images in order from the top of the image request stack. In general, it is desirable for the image requests in the image request stack to be fulfilled quickly rather than introducing the complexity and resulting inefficiency of prioritizing some image requests over others. In some implementations, however, one or more images in the image stack are prioritized over the other images in the stack. For example, an image request for an image that is currently being displayed to a user may be prioritized over other images in the stack. After block 502, process 500 continues to block 504.

At block 504, image requests in the image request stack are added to a queue. In general, the queue stores image requests to be fulfilled. In some embodiments, the queue is the image request stack. The queue may be any data structure and is not limited to a queue data structure. After block 504, process 500 continues to block 506.

At block 506, a determination is made whether there is an image request in the queue. If there is not an image request in the queue, process 500 ends at an end block. If there is an image request in the queue, process 500 continues to block 508.

At block 508, the image request is dequeued. After block 508, process 500 continues to block 510.

At block 510, image data is requested from a server using the image request. In some implementations, when an error is received from the medical image service, the image data is requested again after a selected time elapses. For example, if a 500-level error is received from the medical image service in response to the request for image data, the request for image data may be repeated after 1, 2, or 5 seconds, for example. The request for image data may be resent a configurable number of times, such as 3 or 5 times, when consecutive errors are received. The selected amount of time to wait before requesting the data again may increase after each subsequent retry. An additional random amount of time (i.e., "jitter") may be added to the selected amount of time before retrying, so that retries do not create load spikes on the medical image service. After block 510, process 500 continues to block 512.

At block 512, the image data is received and cached at the client device. After block 512, process 500 continues to block 506, where it is determined whether there is an image request remaining in the queue. If there is an image request remaining in the queue, blocks 508, 510, and 512 are repeated for the image request remaining in the queue.

In some implementations, a configurable number of worker threads are used to request images from the medical image service using the image requests in the queue. The worker threads are typically created on the client device, such that the worker threads may request images to be displayed on the client device. This may enable multithreaded parallel processing of the image request stack, improving performance. For example, several worker threads may request image requests from the queue and use the image requests to request image data from the medical image service.

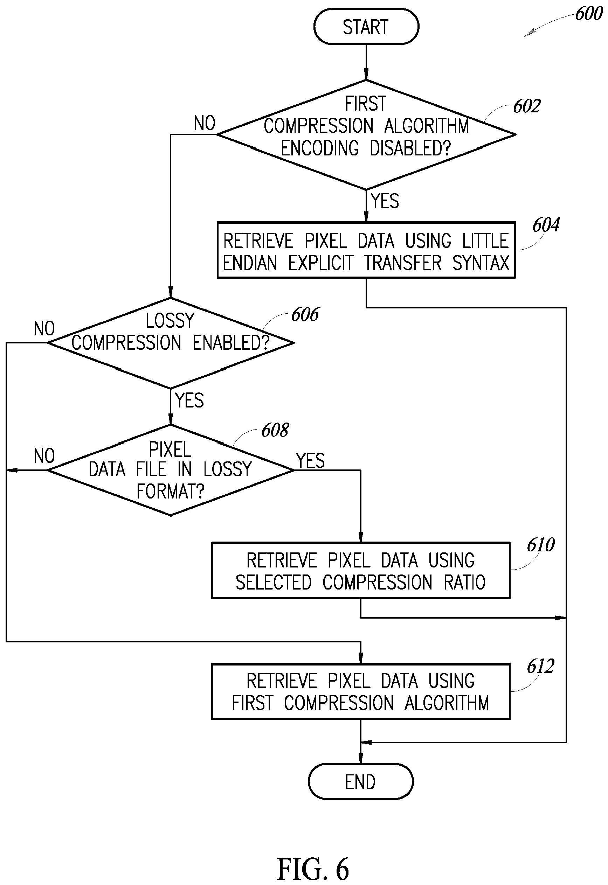

Image data received from the medical image service may be compressed using one of several image compression schemes. Thus, pixel data is extracted from the images received from the medical image service to ensure the images are displayed consistently, as discussed with respect to Figure 6.

Figure 6 is a flow diagram that shows a process 600 for retrieving pixel data from images received from a medical image service in accordance with embodiments described herein. In some implementations, the images are DICOM images. The pixel data that is returned from each image source includes varying levels of processing and compression. In some implementations, a Multipurpose Internet Mail Extension (MIME) type of the image may indicate a relevant compression algorithm for the image.

Process 600 begins, after a start block, at block 602, where a determination is made whether a first compression algorithm encoding is disabled for an image. In some implementations, an attribute of the image such as the image's MIME type may be used to determine whether the first compression algorithm encoding is enabled, and other information regarding compression of the image. In some implementations, a combination of reversible and irreversible compression is used depending on a state of feature flags, a modality of the image, a pixel type of the uncompressed image, or a combination thereof.

The first compression algorithm may be JPEG 2000 reversible compression, JPEG 2000 irreversible compression, etc. JPEG 2000 is an image compression algorithm designed to be a successor to the well-known JPEG compression algorithm. The JPEG 2000 algorithm may have several benefits over JPEG which make it more suitable for compression of medical images. For example, JPEG 2000 outputs data with 32 bits per channel, while JPEG only supports 8 bits per channel. JPEG 2000 does not produce blocking artifacts that are commonly found in images compressed using JPEG. JPEG 2000 enables reversible (i.e., "lossless") compression, while JPEG only offers irreversible (i.e., "lossy") compression.

The MIME type of the image may indicate when the first compression is disabled. When the first compression algorithm encoding is disabled, decompression is not required. Thus, when the first compression algorithm encoding is disabled, process 600 continues from block 602 to block 604, where the pixel data of the image is retrieved using little endian explicit transfer syntax. After block 604, process 600 ends at an end block.

If the first compression algorithm encoding is enabled, decompression is performed. Process 600 therefore continues to block 606, where a determination is made whether lossy compression is enabled. For example, lossy compression may be enabled when JPEG irreversible compression is being used with respect to the image. If lossy compression is not enabled, process 600 continues to block 612, where the pixel data is retrieved using the first compression algorithm. In at least some implementations, a codec such as OpenJPEG is used to decode JPEG 2000 images in a browser of the client device. As browsers may be unable to decode JPEG 2000 images natively, a Web Assembly (WASM) that exports a function to decode JPEG 2000 images in the browser may be produced. In some implementations, threading of the decoding is managed by running the function from multiple web workers (e.g., 4 web workers), increasing performance of the decoding without impacting performance for the rest of the application. After block 612, process 600 ends at an end block.

If lossy compression is enabled, process 600 continues from block 606 to block 608, where a determination is made whether pixel data in the image is encoded in a lossy format. If the pixel data in the image is not encoded in a lossy format, process 600 continues to block 612, where the pixel data is retrieved using the first compression algorithm as described herein. After block 612, process 600 ends at an end block.

If the pixel data is encoded in a lossy format, process 600 continues from block 608 to block 610, where the pixel data is retrieved using a selected compression ratio. In at least some embodiments, the compression ratios used are based on the values presented in the following paper: "Standards for Irreversible Compression in Digital Diagnostic Imaging within Radiology," The Canadian Association of Radiologists (2011), which is hereby incorporated by reference in its entirety. After block 610, process 600 ends at an end block.

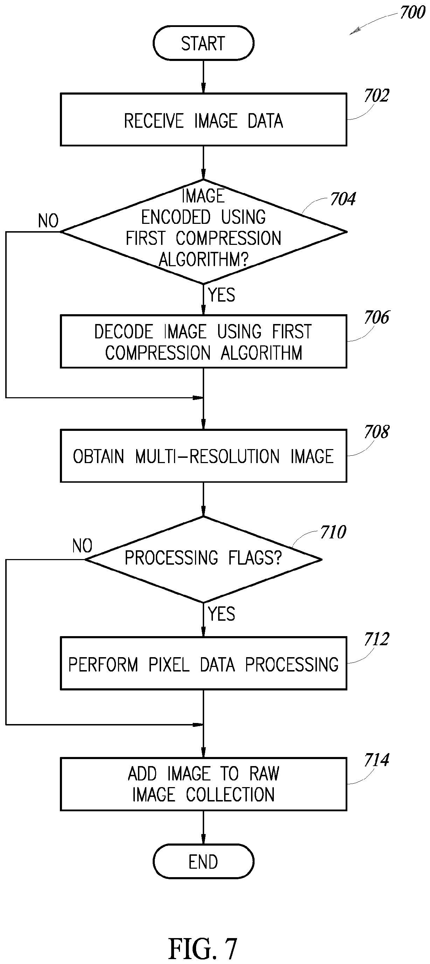

Figure 7 is a flow diagram that shows a process 700 for rendering images in the image viewing pipeline (IVP) according to one non-limiting illustrated implementation. In some implementations, when the image data has been received and decompressed, such as by process 500 and process 600, respectively, the image data may be further processed before being displayed as a GUI component.

At block 702, image data is received. In some implementations, the image data is received using embodiments of process 500 of Figure 5, process 600 of Figure 6, or a combination thereof. After block 702, process 700 continues to block 704.

At block 704, a determination is made whether the image is encoded using a first compression algorithm. If the image is encoded using the first compression algorithm, process 700 continues from block 704 to block 706. If the image is not encoded using the first compression algorithm, process 700 continues to block 708.

At block 706, the image is decoded using the first compression algorithm. In some embodiments, the image is decoded using a WebAssembly module. In some implementations, embodiments of process 600 are employed to decode the image. After block 706, process 700 continues to block 708.

At block 708, a multi-resolution image is obtained. After block 708, process 700 continues to block 710.

At block 710, a determination is made whether there are processing flags associated with the image. In various implementations, processing flags may indicate that any pixel transform specified by the DICOM standard or any other known image processing technique. Examples of pixel transforms that may be specified by processing flags include linear modality transforms specified by DICOM attributes such as RescaleSlope and Rescalelntercept, Image cropping using DICOM attributes such as PercentPhaseFieldOfView and InPlanePhaseEncodingDirection, Look Up Table (LUT), etc. If one or more processing flags are present, process 700 continues to block 712. If no processing flags are present, process 700 continues to block 714.

At block 712, pixel data processing is performed. In various implementations, any transform specified by a processing flag may be performed. In some implementations, the pixel data processing includes window leveling. Window leveling is a grayscale mapping process used to highlight selected features of a medical image. Window leveling is typically controlled using one or more parameters such as a window width and a window level.

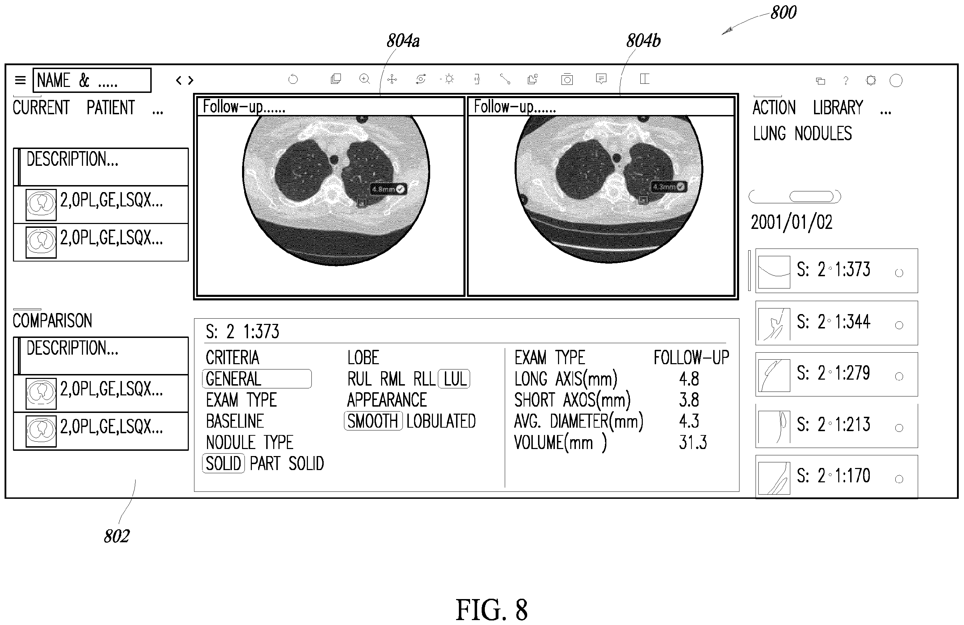

In the case of a CT scan, each pixel in a raw CT scan image corresponds to a certain Hounsfield Unit (HU) value. For example, fat typically has an HU value of around -100, soft tissue typically has an HU value of around 50, bone typically has an HU value of around 400, etc. In general, similar anatomical features have similar HU values. Thus, various soft tissues will typically have HU values in a relatively narrow range such as 50-100 HU and may therefore appear as very similar shades of gray in a raw CT scan image. This is because typically grayscale images only have 256 possible values for each pixel, with black often being 0 and white often being 255. For example, if a raw CT image includes values from -1000 HU to 1000 HU, the range 50-100 HU, which may include various soft tissues, may be represented by a single shade of gray, making the various soft tissues appear the same in the raw CT image. Referring to Figure 8, this can be seen in the first study image 804a that is currently displaying a cross-sectional image of lungs. Air in the lungs appears in dark gray in the images, while the various body tissues and bones surrounding the lungs appear in various lighter shades of gray.

Window width controls how HU values in the raw CT scan image are mapped to a range of grayscale values, such that the CT image may be made sensitive to a selected range of HU values. With a large window width, a relatively wide range of HU values is mapped to the range of grayscale values to be displayed. This allows various anatomical features having different HU values to be displayed in a same grayscale image but decreases contrast between anatomical features with similar HU values. For example, if air and bone are both to be analyzed in the CT scan image, as seen with respect to Figure 8, a relatively wide window may be used.

With a small window width, a relatively narrow range of HU values is mapped to the range of grayscale values, allowing anatomical features having similar HU values to be differentiated from each other. For example, if various soft tissues are to be analyzed in a same grayscale image, a relatively narrow HU window may be used to increase contrast between the various soft tissues.

Window level controls a midpoint in the range of HU levels of the window, defining a cutoff between black pixels and white pixels. Increasing the window level decreases the overall brightness of the image because fewer pixels in the image are mapped to white. Decreasing the window level tends to increase the overall brightness of the image because more pixel values are mapped to white. After block 712, process 700 continues to block 714.

Window leveling is often one of the final operations performed on the pixels before the pixels are displayed as an image. Window leveling may be implemented behind a queue of 1, wherein only a single window leveling command is serviced at a time. Servicing a single command at a time may allow unnecessary memory usage to be reduced by checking whether a window-leveled version of the image is available in the cache. Saving window-leveled images to the cache decreases computing resources used to recompute a window-leveled version of the image. If a window-leveled version of the image is already available in the cache, it does not need to be recom-puted.

At block 714, the processed image is added to a raw image collection. After block 714, process 700 ends at an end block.

Figure 8 shows a screenshot of an example graphical user interface (GUI) 800 for a web-based medical image viewer, in which GUI components 802 and two co-registered studies are presented to a user, in accordance with embodiments described herein.

First study image 804a is presented using a first central image viewer, while second study image 804b is presented using a second central image viewer. Typically, images presented in GUI 800 are obtained using embodiments of process 500, process 600, process 700, or a combination thereof.

First study image 804a and second study image 804b are co-registered, meaning they are images of the same anatomical feature under different conditions, allowing the anatomical feature to be compared under the different conditions. This can be seen in the similarities between the first study image 804a and the second study image 804b. Commonly, the first study and the second study include images taken at a first time and a second time, respectively, allowing changes in the anatomical feature to be compared over time. For example, growth of a tumor may be monitored over time by comparing two or more studies containing images taken at different times.

The client device may display multiple viewports at the same time. In some implementations, the displayed images may be compute-rendered JPEG images. In some implementations, compute JPEG image requests are packaged together, and the client device waits for those requests to complete before rendering the images. However, with the creation of the image viewing pipeline, images are rendered individually as they are available and are not part of the packaged requests.

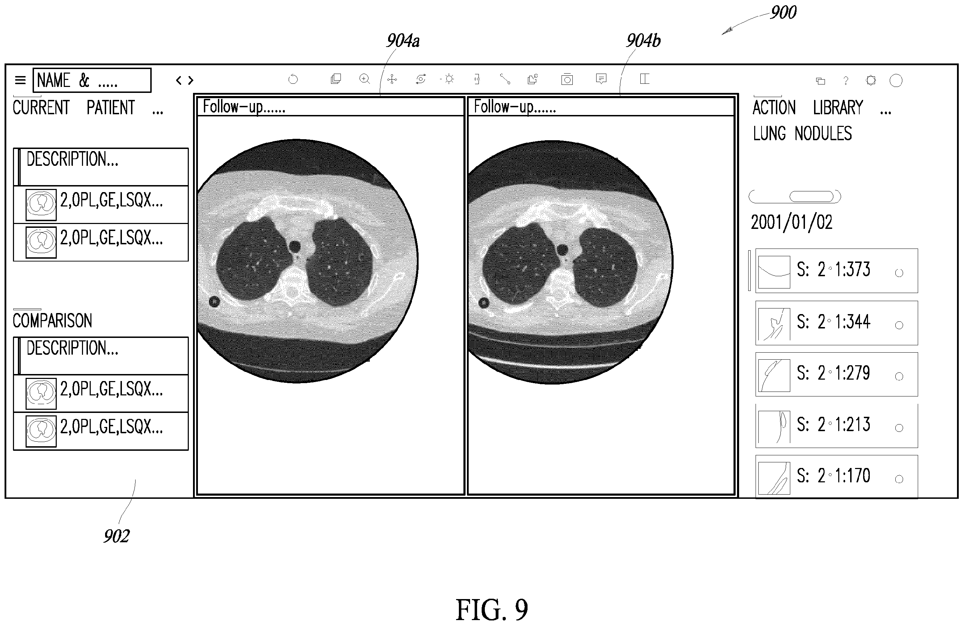

Figure 9 shows a screenshot of an example graphical user interface (GUI) for a web-based medical image viewer, in which two co-registered studies are presented to a user in an enlarged portrait mode, according to one non-limiting illustrated implementation. One or more sizes of first study image 904a and second study image 904b may be configurable in the GUI 900, reducing GUI space used to display other GUI components 902. For example, in Figure 9, first study image 904a and second study image 904b are displayed in enlarged portrait mode.

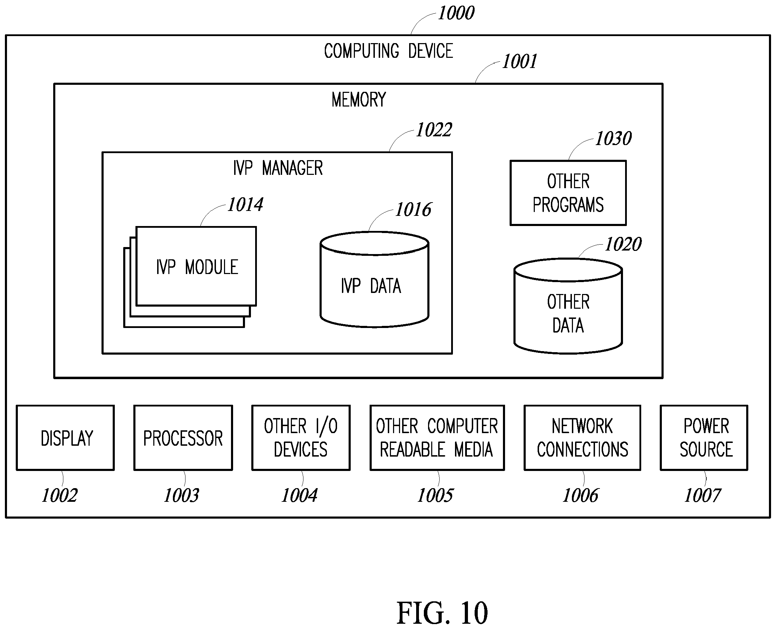

Figure 10 is a block diagram illustrating elements of an example computing device 1000 utilized in accordance with some embodiments of the techniques described herein. Illustratively, the computing device 1000 may correspond to the IVP and related components discussed herein.

In some embodiments, one or more general purpose or special purpose computing systems or devices may be used to implement the computing device 1000. In addition, in some embodiments, the computing device 1000 may comprise one or more distinct computing systems or devices, and may span distributed locations. Furthermore, each block shown in Figure 10 may represent one or more such blocks as appropriate to a specific embodiment or may be combined with other blocks. Also, an IVP manager 1022 may be implemented in software, hardware, firmware, or in some combination to achieve the capabilities described herein.

As shown, the computing device 1000 comprises a computer memory ("memory") 1001, a display 1002 (including, but not limited to a light emitting diode (LED) panel, cathode ray tube (CRT) display, liquid crystal display (LCD), touch screen display, projector, etc.), one or more Central Processing Units (CPU) or other processors 1003, Input/Output (I/O) devices 1004 (e.g., keyboard, mouse, RF or infrared receiver, universal serial bus (USB) ports, High-Definition Multimedia Interface (HDMI) ports, other communication ports, and the like), other computer-readable media 1005, network connections 1006, a power source (or interface to a power source) 1007. The IVP manager 1022 is shown residing in memory 1001. In other embodiments, some portion of the contents and some, or all, of the components of the IVP manager 1022 may be stored on and/or transmitted over the other computer-readable media 1005. The components of the computing device 1000 and IVP manager 1022 can execute on one or more processors 1003 and implement applicable functions described herein. In some embodiments, the IVP manager 1022 may operate as, be part of, or work in conjunction and/or cooperation with other software applications stored in memory 1001 or on various other computing devices. In some embodiments, the IVP manager 1022 also facilitates communication with peripheral devices via the I/O devices 1004, or with another device or system via the network connections 1006.

The one or more IVP modules 1014 are configured to perform actions related, directly or indirectly, to the IVP execution as described herein. In some embodiments, the IVP module(s) 1014 stores, retrieves, or otherwise accesses at least some IVP-related data on some portion of the IVP data storage 1016 or other data storage internal or external to the computing device 1000. In various embodiments, at least some of the IVP modules 1014 may be implemented in software or hardware.

Other code or programs 1030 (e.g., further data processing modules, communication modules, a Web server, and the like), and potentially other data repositories, such as data repository 1020 for storing other data, may also reside in the memory 1001, and can execute on one or more processors 1003. Of note, one or more of the components in Figure 10 may or may not be present in any specific implementation. For example, some embodiments may not provide other computer readable media 1005 or a display 1002.

In some embodiments, the computing device 1000 and IVP manager 1022 include API(s) that provides programmatic access to add, remove, or change one or more functions of the computing device 1000. In some embodiments, components/modules of the computing device 1000 and IVP manager 1022 are implemented using standard programming techniques. For example, the IVP manager 1022 may be implemented as an executable running on the processor(s) 1003, along with one or more static or dynamic libraries. In other embodiments, the computing device 1000 and IVP manager 1022 may be implemented as instructions processed by a virtual machine that executes as one of the other programs 1030. In general, a range of programming languages known in the art may be employed for implementing such example embodiments, including representative implementations of various programming language paradigms, including but not limited to, object-oriented (e.g., Java, C++, C#, Visual Basic.NET, Smalltalk, and the like), functional (e.g., ML, Lisp, Scheme, and the like), procedural (e.g., C, Pascal, Ada, Modula, and the like), scripting (e.g., Perl, Ruby, Python, JavaScript, VBScript, and the like), or declarative (e.g., SQL, Prolog, and the like).

In a software or firmware implementation, instructions stored in a memory configure, when executed, one or more processors of the computing device 1000 to perform the functions of the IVP manager 1022. In some embodiments, instructions cause the one or more processors 1003 or some other processor(s), such as an I/O controller/processor, to perform at least some functions described herein.

The embodiments described above may also use well-known or other synchronous or asynchronous client-server computing techniques. However, the various components may be implemented using more monolithic programming techniques as well, for example, as an executable running on a single CPU computer system, or alternatively decomposed using a variety of structuring techniques known in the art, including but not limited to, multiprogramming, multithreading, client-server, or peer-to-peer, running on one or more computer systems each having one or more CPUs or other processors. Some embodiments may execute concurrently and asynchronously, and communicate using message passing techniques. Equivalent synchronous embodiments are also supported by a IVP manager 1022 implementation. Also, other functions could be implemented and/or performed by each component/module, and in different orders, and by different components/modules, yet still achieve the functions of the computing device 1000 and IVP manager 1022.

In addition, programming interfaces to the data stored as part of the computing device 1000 and IVP manager 1022, can be available by standard mechanisms such as through C, C++, C#, and Java APIs; libraries for accessing files, databases, or other data repositories; scripting languages such as XML; or Web servers, FTP servers, NFS file servers, or other types of servers providing access to stored data. The IVP data storage 1016 and data repository 1020 may be implemented as one or more database systems, file systems, or any other technique for storing such information, or any combination of the above, including implementations using distributed computing techniques.

Different configurations and locations of programs and data are contemplated for use with techniques described herein. A variety of distributed computing techniques are appropriate for implementing the components of the illustrated embodiments in a distributed manner including but not limited to TCP/IP sockets, RPC, RMI, HTTP, and Web Services (XML-RPC, JAX-RPC, SOAP, and the like). Other variations are possible. Other functionality could also be provided by each component/module, or existing functionality could be distributed amongst the components/modules in different ways, yet still achieve the functions of the IVP manager 1022.

Furthermore, in some embodiments, some or all of the components of the computing device 1000 and IVP manager 1022 may be implemented or provided in other manners, such as at least partially in firmware and/or hardware, including, but not limited to one or more application-specific integrated circuits ("ASICs"), standard integrated circuits, controllers (e.g., by executing appropriate instructions, and including microcontrollers and/or embedded controllers), field-programmable gate arrays ("FPGAs"), complex programmable logic devices ("CPLDs"), and the like. Some or all of the system components and/or data structures may also be stored as contents (e.g., as executable or other machine-readable software instructions or structured data) on a computer-readable medium (e.g., as a hard disk; a memory; a computer network, cellular wireless network or other data transmission medium; or a portable media article to be read by an appropriate drive or via an appropriate connection, such as a DVD or flash memory device) so as to enable or configure the computer-readable medium and/or one or more associated computing systems or devices to execute or otherwise use, or provide the contents to perform, at least some of the described techniques.

The following is a summary of the claims as originally filed.

A method may be summarized as including: hosting, by a first server, first graphical user interface (GUI) components of a GUI of a web-based medical viewer application, the first GUI components comprising images to be presented in the GUI of the web-based medical viewer application; hosting, by a second server, second GUI components of the GUI, the second GUI components comprising GUI components other than the first GUI components to be presented in the GUI of the web-based medical viewer application; serving the second GUI components from the second server to a client device for rendering the GUI to a user of the client device; detecting, by the second server, interaction with a component in the second GUI components; authenticating the client device to receive the first GUI components; receiving, by the first server and from the client device, selection of a medical study; and in response to receiving the selection of the medical study, serving the first GUI components from the first server to the client device for rendering the GUI to the user of the client device.

In some implementations, at least one of the first server and the second server may be implemented using a container.

In some implementations, serving the first GUI components from the first server to the client device for rendering the GUI to the user of the client device includes: serving compressed image data to the client device.

In some implementations, the method further includes: running multiple concurrent versions of the first server simultaneously; and routing a configurable proportion of traffic to a version of the first server in the multiple concurrent versions of the first server.

In some implementations, the method further includes: running multiple concurrent versions of the first server behind a load balancer; and routing, by the load balancer, a configurable proportion of traffic to a version of the first server in the multiple concurrent versions of the first server.

In some implementations, serving the first GUI components from the first server to the client device for rendering the GUI to the user of the client device includes: serving, by the first server, an image that the user is currently attempting to view; and after serving the image that the user is currently attempting to view, serving, by the first server, images in order from an image request stack.

In some implementations, serving the first GUI components from the first server to the client device for rendering the GUI to the user of the client device includes: serving, by the first server, the first GUI components that include a plurality of co-registered sets of medical images to be concurrently displayed to the user of the client device.

In some implementations, the method further includes: updating the second server while providing, to the client device and by the first server, the first GUI components.

In some implementations, the method further includes: obtaining, by the first server, an image request stack for the images to be presented in the GUI of the web-based medical viewer application; selecting, by the first server, an image request from the image request stack; requesting, by the first server, image data from a medical image service using the selected image request; and receiving and caching, by the first server, the image data.

In some implementations, the method further includes: causing the client device to retrieve pixel data of an image of the images to be presented in the GUI of the web-based medical viewer application using a first compression algorithm and a selected compression ratio in response to determining that: the image is compressed using lossy compression.

In some implementations, the method further includes: determining that image data of a selected image of the images to be presented in the GUI of the web-based medical viewer application includes a processing flag that specifies a transform to be applied to the image data; and causing the client device to apply the specified transform to the image data.

In some implementations, the method further includes: determining that a window-leveled version of a selected image in the images to be presented in the GUI of the web-based medical viewer application is not stored in a cache available to the client device; and creating the window-leveled version of the selected image by causing the client device to perform actions including: obtaining a window width and a window level to be used in creating the window-leveled version of the selected image; and creating the window-leveled version of the selected image by modifying pixels in the selected image using the window width and the window level.

In some implementations, the method further includes: detecting, by the client device, an error in receiving the first GUI components from the first server; and in response to detecting the error, requesting the first GUI components from the second server.

A computing system may be summarized as including: one or more processors; and one or more non-transitory computer-readable media collectively storing instructions that, when collectively executed by the one or more processors, cause the one or more processors to perform the method.

A non-transitory computer-readable medium may be summarized as being configured to store computer instructions that, when collectively executed by one or more processors, cause the one or more processors to perform the method.

The various embodiments described above can be combined to provide further embodiments. All of the U.S. patents, U.S. patent application publications, U.S. patent applications, foreign patents, foreign patent applications and non-patent publications referred to in this specification and/or listed in the Application Data Sheet, including U.S. Provisional Application No. 63/492,350 filed on March 27, 2023, are incorporated herein by reference, in their entirety. Aspects of the embodiments can be modified, if necessary to employ concepts of the various patents, applications, and publications to provide yet further embodiments.

These and other changes can be made to the embodiments in light of the above-detailed description. In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.

## Claims

1. A method, comprising:
hosting, by a first server (212), first graphical user interface (GUI) components (104) of a GUI of a web-based medical viewer application, the first GUI components comprising images to be presented in the GUI of the web-based medical viewer application;
hosting, by a second server (202), second GUI components (102) of the GUI, the second GUI components comprising GUI components other than the first GUI components to be presented in the GUI of the web-based medical viewer application;
serving the second GUI components from the second server to a client device for rendering the GUI to a user of the client device;
detecting, by the second server, interaction with a component in the second GUI components;
authenticating the client device to receive the first GUI components;
receiving, by the first server and from the client device, selection of a medical study; and
in response to receiving the selection of the medical study, serving the first GUI components from the first server to the client device for rendering the GUI to the user of the client device.

2. The method of claim 1, wherein at least one of the first server and the second server is implemented using a container.

3. The method of claim 1 or claim 2, wherein serving the first GUI components from the first server to the client device for rendering the GUI to the user of the client device comprises:
serving compressed image data to the client device.

4. The method of any one of claims 1-3, further comprising:
running multiple concurrent versions of the first server simultaneously; and
routing a configurable proportion of traffic to a version of the first server in the multiple concurrent versions of the first server.

5. The method of any one of claims 1-4, further comprising:
running multiple concurrent versions of the first server behind a load balancer (214); and
routing, by the load balancer, a configurable proportion of traffic to a version of the first server in the multiple concurrent versions of the first server.

6. The method of any one of claims 1-5, wherein serving the first GUI components from the first server to the client device for rendering the GUI to the user of the client device comprises:
serving, by the first server, an image that the user is currently attempting to view; and
after serving the image that the user is currently attempting to view, serving, by the first server, images in order from an image request stack.

7. The method of any one of claims 1-6, wherein serving the first GUI components from the first server to the client device for rendering the GUI to the user of the client device comprises:
serving, by the first server, the first GUI components that include a plurality of co-registered sets of medical images to be concurrently displayed to the user of the client device.

8. The method of any one of claims 1-7, further comprising:
updating the second server while providing, to the client device and by the first server, the first GUI components.

9. The method of any one of claims 1-8, further comprising:
obtaining, by the first server, an image request stack for the images to be presented in the GUI of the web-based medical viewer application;
selecting, by the first server, an image request from the image request stack;
requesting, by the first server, image data from a medical image service using the selected image request; and
receiving and caching, by the first server, the image data.

10. The method of any one of claims 1-9, further comprising:
causing the client device to retrieve pixel data of an image of the images to be presented in the GUI of the web-based medical viewer application using a first compression algorithm and a selected compression ratio in response to determining that:
the image is compressed using lossy compression.

11. The method of any one of claims 1-10, further comprising:
determining that image data of a selected image of the images to be presented in the GUI of the web-based medical viewer application includes a processing flag that specifies a transform to be applied to the image data; and
causing the client device to apply the specified transform to the image data.

12. The method of any one of claims 1-11, further comprising:
determining that a window-leveled version of a selected image in the images to be presented in the GUI of the web-based medical viewer application is not stored in a cache available to the client device; and
creating the window-leveled version of the selected image by causing the client device to perform actions comprising:
obtaining a window width and a window level to be used in creating the window-leveled version of the selected image; and
creating the window-leveled version of the selected image by modifying pixels in the selected image using the window width and the window level.

13. The method of any one of claims 1-12, further comprising:
detecting, by the client device, an error in receiving the first GUI components from the first server; and
in response to detecting the error, requesting the first GUI components from the second server.

14. A computing system comprising:
one or more processors; and
one or more non-transitory computer-readable media collectively storing instructions that, when collectively executed by the one or more processors, cause the one or more processors to perform the method of any one of claims 1 to 13.

15. A non-transitory computer-readable medium that stores computer instructions that, when collectively executed by one or more processors, cause the one or more processors to perform the method of any one of claims 1 to 13.
